(19) Europäisches Patentamt
European Patent Office
Office européen des brevets

(11) Publication number: **0 482 594 A1**

# (12) EUROPEAN PATENT APPLICATION

(21) Application number: **91118020.6**

(51) Int. Cl.⁵: **C07H 17/08**, A61K 31/70

(22) Date of filing: **23.10.91**

(30) Priority: **25.10.90 JP 288242/90**

(43) Date of publication of application:
**29.04.92 Bulletin 92/18**

(84) Designated Contracting States:
**DE ES FR GB IT**

(71) Applicant: **MERCIAN CORPORATION**
**5-8, Kyobashi 1-chome**
**Chuo-ku, Tokyo(JP)**

(72) Inventor: **Yoshioka, Takeo**
**3-3102, Green-Haitsu, 1959, Kamitsuchidana**
**Ayase-shi, Kanagawa-ken(JP)**
Inventor: **Kominato, Kaichiro**
**6-4-30, Minami-Rinkan**
**Yamato-shi, Kanagawa-ken(JP)**
Inventor: **Sakamoto, Michiko**
**3-7-15, Shorin**
**Chigasaki-shi, Kanagawa-ken(KP)**
Inventor: **Tanaka, Hiroshi**
**3-16-26-306, Kowada**
**Chigasaki-shi, Kanagawa-ken(KP)**
Inventor: **Tone, Hiroshi**
**3-7-4-1003, Namiki, Kanazawa-ku**
**Yokohama-shi, Kanagawa-ken(KP)**
Inventor: **Okamoto, Rokuro**
**2-18, Hananoki**
**Fujisawa-shi, Kanagawa-ken(KP)**
Inventor: **Sawa, Tsutomu**
**4-6-7, Ryosei**
**Ayase-shi, Kanagawa-ken(KP)**
Inventor: **Takeuchi, Tomio**
**5-1-11, Higashi-Gotanda**
**Shinagawa-ku, Tokyo(KP)**

(74) Representative: **Werner, Hans-Karsten et al**
**Patentanwälte von Kreisler Selting Werner**
**Deichmannhaus am Hauptbahnhof**
**W-5000 Köln 1(DE)**

## (54) Tylosin derivative and method for preparing the same.

(57) The compound 4''-O-p-Methoxymandeloyltylosin. The compound has excellent antibacterial activity and a reduced inhibitory activity on animal cells. Also are a method for preparing the compound, a pharmaceutical composition comprising the compound, and a method for treating an infectious disease comprising administering the compound.

EP 0 482 594 A1

## BACKGROUND OF THE INVENTION

### Field of the Invention

The present invention relates to a novel derivative of tylosin, a macrolide antibiotic, and a method for preparing the same.

### Description of the Related Art

Tylosin is a macrolide antibiotic which has been widely used for the treatment of infectious diseases of animals and as a feed additive. Various kinds of tylosin derivatives, which have higher antibacterial activity and improved administration and elimination properties in vivo, are described in patent documents such as, for example, the Japanese Patent Unexamined Publication (hereinafter KOKOKU) Nos. 53-137982 and 54-70291, and the Japanese Patent Publication (hereinafter KOKOKU) No. 55-23272.

Tylosin derivatives, which have sufficient antibacterial activity against macrolide-resistant bacteria and are resistant to hydrolysis action of esterases in a living body, are described in recent patent documents such as, for example, the Japanese KOKAI Nos. 60-1198, 61-30596, and 61-137895.

However, besides inhibiting the growth of microorganisms, these tylosin derivatives have the disadvantage of inhibiting the growth or the colony formation of some kinds of animal cells, and thus, the elimination of this disadvantage has been desired from the standpoint of selective toxicity of antibiotics.

## SUMMARY OF THE INVENTION

An object of the present invention is to provide a tylosin derivative having potent antibacterial activity against various kinds of microorganisms and a reduced inhibitory activity against the growth of animal cells.

Another object of the present invention is to provide a method for preparing said tylosin derivative.

Further objects of the present invention are to provide a pharmaceutical composition comprising an effective amount of said tylosin derivative together with a pharmaceutically-acceptable carrier or coating.

Yet another object of the present invention is to provide a method for treating an infectious disease of an animal comprising the step of administering an effective amount of said tylosin derivative or pharmaceutical composition.

The inventors of the present invention have conducted various studies to achieve the foregoing objects and found that the objects can be effectively attained by providing a tylosin derivative which has a para-methoxymandeloyl group at the 4"-position. The inventors have also found that the tylosin derivative has excellent antibacterial activity and induces highly reduced inhibition of the growth or the colony formation of animal cells.

Thus, in accordance with the above objects, the present invention provides a tylosin derivative represented by the following formula:

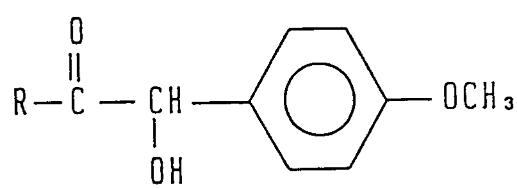

wherein R is a group represented by the following formula.

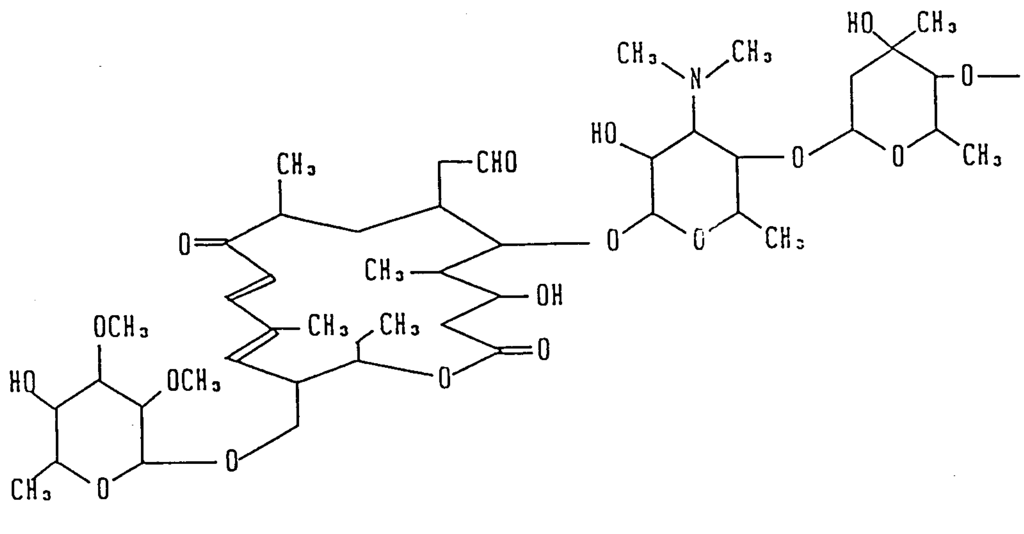

In accordance with another embodiment of the present invention, there is provided a process for preparing said tylosin derivative comprising the steps of (a) condensing a hydroxy-protected para-methoxymandelic acid with tylosin or a tylosin derivative having one or more protective groups; and (b) removing the protective group to obtain 4"-O-p-methoxymandeloyltylosin.

In accordance with yet another embodiment, the present invention provides a pharmaceutical composition comprising an effective amount of said tylosin derivative together with a pharmaceutically-acceptable carrier or coating.

In accordance with a still further embodiment, the present invention provides a method for treating an infectious disease of an animal comprising the step of administering an effective amount of said tylosin derivative or the pharmaceutical composition comprising the same.

Further objects, features and advantages of the present invention will become apparent from the Description of the Preferred Embodiments which follows, when read in light of the accompanying Examples.

## DETAILED DESCRIPTION OF THE PREFERRED EMBODIMENTS

The tylosin derivative of the present invention has a paramethoxymandeloyl substituent at the 4"-position of tylosin. The tylosin derivative of the present invention includes (-)-para-methoxymandeloyl derivatives, (+)-para-methoxymandeloyl derivatives, mixtures of the optically active para-methoxymandeloyl derivatives, and racemic para-methoxymandeloyl derivatives, i.e., (±)-para-methoxymandeloylderivatives which contain an equal amount of (-)-para-methoxymandeloyl derivatives and (+)-para-methoxymandeloyl derivatives.

The preparation of the tylosin derivative of the present invention may be conducted according to the method of preparing the tylosin derivative which comprises the steps of:

(a) condensing a hydroxy-protected para-methoxymandelic acid with tylosin or a tylosin derivative having one or more protective groups; and

(b) removing the protective group to obtain 4"-O-p-methoxymandeloyltylosin.

para-Methoxymandelic acid may optionally be resolved according to a known procedure such as, for example, the resolution process described in the Japanese KOKAI No. 53-115988 or the process described in Example 1 of the present specification. The latter process includes the steps of preparing the salt of racemic para-methoxymandelic acid with (S)-(-)-α-methylbenzylamine, and separating the resulting diastereomeric salts by a physico-chemical procedure such as, for example, recrystallization, to obtain an optically active para-methoxymandelic acid.

Any suitable protective group such as those well known to those skilled in the art may be used as the protective group for the hydroxyl group of p-methoxymandelic acid. Examples of the hydroxy-protecting groups include, for example, a chloroacetyl group, a dichloroacetyl group, a trichloroacetyl group, a bromoacetyl group, and a tetrahydropyranyl group. An example of the protected tylosin having one or more protective groups is 2'-O-acetyl-4'''-O-trimethylsilyltylosin described in the Japanese KOKAI No. 1-317495.

The condensation reaction may generally be carried out at a temperature in the range of from -30 to 25°C for 30 to 180 minutes in the presence of a condensing agent such as, for example, pyridine-pivaloyl chloride in a solvent such as, for example, dichloromethane or dichloroethane. The tylosin derivative of the

present invention can be obtained by removing the protective groups of the condensate, obtained in the above-described manner, according to any one of deprotecting reactions known to one having ordinary skill in the art.

Antibacterial activity

Antibacterial activity of the tylosin derivative of the present invention was determined by the agar-dilution method using a Mueller Hinton agar medium (Difco). The results obtained are summarized in Table 1 in which minimum inhibitory concentrations (MICs, µg/ml) are shown. Hereinafter in the specification, Compound A (the compound described in Example 3) is defined as being 4"-O-[(S)-(+)-p-methoxymandeloyl]tylosin represented by the following formula:

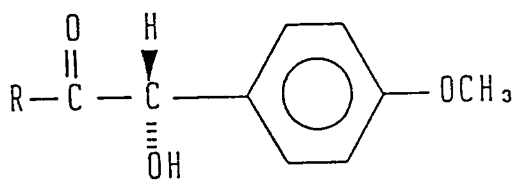

wherein R is the same as that defined above ;
Compound B (the compound described in Example 2) is defined as being 4"-O-[(R)-(-)-p-methoxymandeloyl]tylosin represented by the following formula:

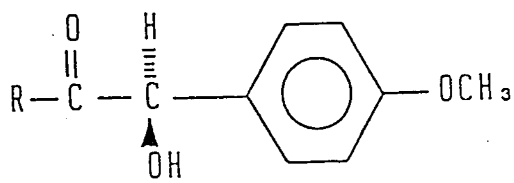

wherein R is the same as that defined above;
XV represents 4"-O-[p-methoxyphenylacetyl]tylosin;
RKM represents rokitamycin; and
EM represents erythromycin.

Table 1

| Antibacterial activity | | | | | |
|---|---|---|---|---|---|
| Bacterial Strain | A | B | XV | RKM | EM |
| Staphylococcus aureus 209P | 0.39 | 0.39 | 0.20 | 0.20 | 0.10 |
| Smith | 1.56 | 1.56 | 1.56 | 0.39 | 0.20 |
| ML-resistant strain * | 6.25 | 6.25 | 6.25 | > 100 | > 100 |
| ML-resistant strain * | 0.39 | 0.39 | 0.78 | > 100 | > 100 |
| ML-resistant strain * | 6.25 | 3.13 | 3.13 | > 100 | > 100 |
| ML-resistant strain * | < 0.20 | < 0.20 | 0.39 | < 0.20 | > 100 |
| ML-resistant strain * | 6.25 | 1.56 | 1.56 | > 100 | > 100 |
| Staphylococcus epidermidis ATCC 12228 | 0.39 | 0.78 | 0.78 | < 0.20 | < 0.20 |
| Bacillus subtilis ATCC 6633 | 0.39 | 0.39 | 0.39 | 0.10 | 0.10 |
| Escherichia coli K12 | > 100 | > 100 | > 100 | 100 | 50 |

* ML-resistant strain: macrolide resistant strain

Stability test using mouse liver homogenate

A liver in collected from an ICR mouse was homogenized by a Potter homogenizer in five times volume of 0.1M phosphate buffer solution (pH 7.2), and supernatant was obtained by centrifuging the homogenate at 3000 rpm for 10 minutes. To 2 ml of the supernatant obtained above, 2 ml of sample solution containing 500 µg/ml of test compound in 20% aqueous methanol and 1 ml of 0.1 M phosphate buffer solution were added, and the mixture was incubated at 37 °c for 60 minutes and then 1 ml of the mixture was heated at 85 °C for 3 minutes. The reaction mixture was adjusted to pH 9.0 by adding 1N NaOH and extracted with 1 ml of ethyl acetate. The organic layer was applied to a slica gel thin layer chromatography (developer: n-hexane/benzene/acetone/ethyl acetate/methanol = 150/125/50/100/40), and the percent ratio of unhydrolyzed substance was calculated using the value representing the ratio between the remaining unhydrolyzed substance and hydrolyzed subtance determined by chromatoscanner (282 nm). The results are summarized in Table 2.

Table 2

| Remaining unhydrolyzed substance (%) after being treated with liver homogenate for 60 minutes. | |
|---|---|
| Compound | unhydrolyzed (%) |
| A | 97.8 |
| B | 100 |
| XV | 92.8 |

Inhibitory activity on the growth of L1210 cells

Mouse leukemia L1210 cells were cultured in RPMI1640 medium (Gifco) containing 10% horse serum (Gifco) at 37°C under 5% carbon dioxide gas so that the leukemia cells were contained in the amount of 5 x $10^4$ cells/ml, and the cells were subjected to passage culture every two or three days to maintain the cell density of 5 x $10^4$ cells/ml. The diluted sample solution (0.1 ml) was added to each well of a 24-well plate which contained 0.9 ml of the cultured L1210 cells, and the mixture was incubated at 37 °C for 48 hours under 5% carbon dioxide gas. The sample solution mentioned above was prepared by dissolving a weighed test compound in dimethyl sulfoxide (DMSO) at the concentration of 100 mg/ml and then diluting the solution with distilled water so that the concentration became 10 mg/ml. The resulting aqueous solution was diluted with phosphate buffer solution to obtain a sample solution at a desired concentration. After incubation, the cell numbers in each well were counted and inhibitory activity on cell growth ($IC_{50}$) was calculated. The results are shown on Table 3.

Table 3

| Inhibitory activity on cell growth (%) | | | |
|---|---|---|---|
| Concentration of derivatives (µg/ml) | XV | A | B |
| 1 | 12.22 | 0 | 0 |
| 3 | 31.18 | 0 | 0 |
| 10 | 82.87 | 5.62 | 13.48 |
| 30 | 90.45 | 47.75 | 41.43 |
| 100 | 93.68 | 88.76 | 87.22 |
| $IC_{50}$ (µg/ml) | 5.01 | 36.62 | 32.99 |

Inhibition of the colony formation of mouse bone marrow cells

The colony forming test was conducted according to the method described by Kodama et al. [Blood, Vol.57, 1119, (1981)] . sample solutions at a concentration of 1 μg/ml were used, and the ratio of colony formation was calculated by the following formula:

$$\text{Ratio of colony formation} = \frac{\text{The number of colonies in the presence of drug}}{\text{The number of colonies in the absence of drug (control)}} \times 100$$

The results are summarized in Table 4

Table 4

Inhibition of the colony formation of mouse bone marrow cells

| Compound | Conc. (μg/ml) | Number of colonies 1 | 2 | 3 | Average | Colony formation (%) |
|---|---|---|---|---|---|---|
| Control | 0 | 95 | 66 | 65 | 75.3 | 100 |
| XV | 1.0 | 13 | | | 13.0 | 17.3 |
| Rokitamycin | 1.0 | | | 19 | 19.0 | 25.2 |
| A | 1.0 | 32 | 30 | | 31.0 | 41.2 |
| B | 1.0 | 34 | 25 | | 29.5 | 39.2 |

From the results shown above, one of ordinary skill in the art will recognize that the tylosin derivative of the present invention has excellent antibacterial activity and a reduced inhibition on animal cells, and that the tylosin derivative of the present invention has an advantageous selective toxicity compared with tylosin or known tylosin derivatives. It will also be recognized that the tylosin derivative is quite useful for the treatment of infectious diseases in animals.

The tylosin derivative of the present invention may be administered orally or parenterally to an animal, preferably as a pharmaceutical composition which comprises an effective amount of said compound together with a pharmaceutically acceptable carrier or coating.

The pharmaceutical composition suitable for oral subtilized granules, granules, solution, or syrup. The pharmaceutical composition suitable for parenteral administration may be injection, suppository, inhalant, eye drop, nasal drop, ointment, or cataplasm. The pharmaceutically acceptable carrier or coating used for the preparation of the pharmaceutical composition may be, for example, excipient, disintegrant or agent for accelerating disintegration, binder, lubricant, coating agent, pigment, diluent, base, solubilizing agent or solubilizer, isotonicity agent, pH adjusting agent, stabilizer, propellant, or adhesive.

For the preparation of the pharmaceutical composition suitable for oral administration, dermal administration, or mucosal application, the coating or carrier may comprise the following: an excipient such as, for example, glucose, lactose, D-mannitol, starch, or crystalline cellulose; a disintegrant or an agent for accelerating disintegration such as, for example, carboxymethyl cellulose, starch, or calcium carboxymethyl cellulose; a binder such as, for example, hydroxypropyl cellulose, hydroxypropylmethyl cellulose, polyvinyl pyrrolidone, or gelatin; a lubricant such as, for example, magnesium stearate or talc; a coating agent such as, for example, hydroxypropylmethyl cellulose, sucrose, polyethylene glycol, or titanium oxide; a base such as, for example, petrolatum, liquid paraffin, polyethylene glycol, gelatin, kaolin, glycerin, purified water,

or hard fat; a propellant such as, for example, freon, diethylether, or compressed gas; an adhesive such as, for example, sodium polyacrylate, polyvinyl alcohol, methylcellulose, polyisobutylene, or polybutene; or a base sheet such as, for example, cloth or plastic sheet. The pharmaceutical composition suitable for injection may comprise the following: a solubilizing agent or a solubilizer, e.g., distilled water for injection, saline, or propylene glycol which is useful for an aqueous composition or a composition for preparing aqueous solution before use; an isotonicity agent such as, for example, glucose, sodium chloride, D-mannitol, or glycerin; and a pH adjusting agent such as, for example, an inorganic or organic acid or an inorganic or organic base.

The dose of the pharmaceutical composition of the present invention for an adult patient may generally be from about 500 to 2000 mg per day for oral administration, which may be increased or decreased depending on the age or condition of the patient to be treated.

The present invention will be further illustrated by the following Examples. The Examples are given by way of illustration only and are not to be construed as limiting.

Example 1: Optical resolution of (±)-p-methoxymandelic acid

(±)-p-Methoxymandelic acid (1.04g, 5.69 mmol) was dissloved in 32 ml of 2-propanol/water (9/1: v/v), and 0.73 ml of (S)-(-)-α-methylbenzylamine (5.72 mmol) was added to the solution and precipitated salts were dissolved by heating. The solution was cooled to 5°C, and then the salts precipitated were collected by filtration. The salts obtained were dissolved in 29 ml of 2-propanol/water (9/1: v/v) under heating, and then the solution was cooled to 5 °C and the precipitated salts were saparated to give 622 mg of (S)-(+)-p-methoxymandelic acid • (S)-(-)-α-methylbenzylamine salt (yield: 76.7% based on (S)-(+)-p-methoxymandelic acid contained in the (±)-p-methoxymandelic acid used).

$[\alpha]_D^{26}$ +60.1° (c 1.0, water)

Water (50 ml) and 1N HCl (1.8 ml) were added to 500 mg of the (S)-(+)-p-methoxymandelic acid • (S)-(-)-α-methylbenzylamine salt (1.65 mmol), and the resulting solution was extracted with ethyl acetate. The organic layer was dried over anhydrous sodium sulfate and the solvent was evaporated under reduced pressure to give 277 mg of (S)-(+)-p-methoxymandelic acid (resolving yield: 70.9%).

$[\alpha]_D^{24}$ +140.1 ° (c 0.3, water)

The primary mother liquor, obtained by separating the (S)-(-)-α-methylbenzylamine salt, was evaporated under reduced pressure, and the residue was dissolved in 20 ml of water. To the solution, 3 ml of 1N aqueous HCl was added and the resulting solution was extracted with ethyl acetate. The organic layer was dried over anhydrous sodium sulfate and the solvent was evaporated under reduced pressure to give 522 mg of (R)-(-)-p-methoxymandelic acid (optical purity: 84%, HPLC method).

The (R)-(-)-p-methoxymandelic acid (460 mg, 2.52 mmol) obtained above was dissolved in 22.5 ml of 2-propanol/water (9/1: v/v), and 0.325 ml of (R)-(+)-α-methylbenzylamine (2.55 mmol) was added to the solution and the salts precipitated were dissolved by heating. The solution was cooled to 5°C, and the precipitated salts were separated by filtration to give 590 mg of (R)-(-)-p-methoxymandelic acid • (R)-(-)-α-methylbenzylamine salt (yield: 77.7% based on (R)-(+)-p-methoxymandelic acid contained in the (±)-p-methoxymandelic acid used).

$[\alpha]_D^{25}$ -60.6° (c 1.0, water)

Water (20 ml) and 1N HCl (1.7 ml) were added to 500 mg of (R)-(-)-p-methoxymandelic acid • (R)-(+)-α-methylbenzylamine salt (1.65 mmol), and the resulting solution was extracted with ethyl acetate. The organic layer was dried over anhydrous sodium sulfate and the solvent was evaporated under reduced pressure to give 285 mg of (R)-(-)-p-methoxymandelic acid (resolving yield: 74.1%).

$[\alpha]_D^{26}$ -135.2° (c 0.3, water)

Example 2

(a) Preparation of (R)-(-)-O-chloroacetyl-p-methoxymandelic acid.

(R)-(-)-p-methoxymandelic acid (183 mg, 1.00 mmol) was dissolved in 4 ml of tetrahydrofuran and the solution was cooled to -20 °C. After 0.203 ml of pyridine (2.51 mmol) was added, 0.168 ml of chloroacetyl chloride (2.11 mmol) was gradually added to the solution and the mixture was stirred at -20 °C for 2 hours. Toluene (5 ml) were water (5 ml) was added to the reaction mixture, and then the reaction mixture was warmed up to room temperature. The pH of the reaction mixture was adjusted to 1.5 with 1N HCl to separate the toluene layer, and the toluene layer was washed with water and dried over anhydrous sodium

sulfate, and then the solvent was evaporated under reduced pressure. The residue obtained was chromatographed on a column of 14 g silica gel (eluent: chloroform/methanol = 50/1 :v/v) to give 134 mg of (R)-(-)-O-chloroacetyl-p-methoxymandelic acid (yield: 51.8%).

The physical properties of the title compound are as follows:

$[\alpha]_D^{25}$ -137° (c 1.08, acetone)

$$IR\ \nu_{max}^{CHCl_3}\ (cm^{-1}):$$

1730, 1605, 1580, 1505, 1245, 1160, 1025, 825

$^1$H-NMR ($CDCl_3$, δ ppm):

3.79(3H,s,$OCH_2$), 4.15(2H,ABq,J = 15.2Hz,$COCH_2Cl$), 5.96(1H,s,-$C_6H_4$-CH(OH)-CO), 6.91(2H,d,J = 8.8Hz,

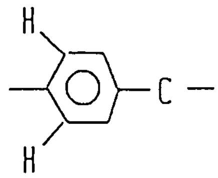

),

7.38(2H,d,J = 8.8Hz,

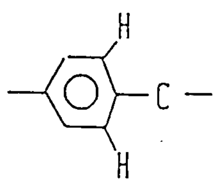

),

9.56(1H,brs, COOH)

(b) Preparation of 2'-O-acetyl-4''-O-[(R)-(-)-O-chloroacetyl-p-methoxymandeloyl]-4'''-O-trimethylsilyltylosin

(R)-(-)-O-Chloroacetyl-p-methoxymandelic acid (108 mg, 0.418 mmol) was dissolved in 1.5 ml of 1,2-dichloroethane. The solution was cooled to -30 °C and then 0.034 ml of pyridine (0.42 mmol) and 0.053 ml of pivaloyl chloride (0.44 mmol) were added to the solution and the mixture was stirred at -30°C. After 20 minutes, 108 mg of 2'-O-acetyl-4'''-O-trimethylsilyltylosin (0.105 mmol), 0.073 ml of triethylamine (0.52 mmol), and 4.4 mg of 4-dimethylaminopyridine (0.036 mmol) were added to the mixture, and stirring was continued at the same temperature for 2.5 hours. A small amount of chloroform and 5 ml of water were added to the reaction mixture, and then the mixuture was warmed to room temperature to separate an organic layer. The separated organic layer was washed with a saturated aqueous sodium bicarbonate and then with a saturated sodium chloride solution, dried over anhydrous sodium sulfate, and the solvent was evaporated. The residue was choromatographed on a column of 10 g silica gel (eluent: toluene/acetone = 8/1 and then 7/1: v/v) to give 29.3 mg of 2'-O-acetyl-4''-O-[(R)-(-)-O-chloroacetyl-p-methoxymandeloyl]-4'''-O-trimethylsilyltylosin (yield: 22.2%).

The physical properties of the title compound are as follows:

m.p. 114.0-117.0°C

$$UV\ \lambda_{max}^{MeOH}\ (nm):$$

281.4(ε 18000), 234.1(ε 11400)

$$IR\ \nu_{max}^{CHCl_3}\ (cm^{-1}):$$

3430, 2920, 1740, 1715, 1675, 1605, 1585, 1510, 1250, 1165, 1095, 875, 840

$^1$H-NMR ($CDCl_3$, $\delta$ ppm):

0.17(9H,s,$(CH_3)_3$Si), 0.65(3H,d,J = 6.2Hz,H-6''), 1.78(3H,s,12-$CH_3$), 2.07(3H,s,2'-OAc), 2.32(6H,s,3'-N$(CH_3)_2$). 3.50(3H,s,2'''-$OCH_3$), 3.60(3H,s,3'''-$OCH_3$), 3.81(3H,s,$CH_3O$-$C_6H_4$-), 5.94(1H,d,J = 10.3Hz,H-13), 6.03(1H,s,-$C_6H_4$-CH(OH)-CO), 6.28(1H,d,J = 15.4Hz,H-10), 6.90(2H,d,J = 8.8Hz,

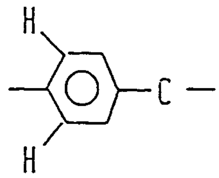

),

7.32(1H, d, J = 15.4Hz, H-11), 7.42(2H,d,J = 8.8Hz,

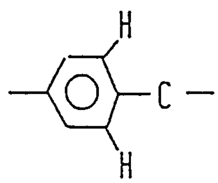

),

9.66(1H,d,J = 1.1Hz, CHO)

(c) Preparation of 4''-O-[(R)-(-)-p-methoxymandeloyl]tylosin (Compound B)

2'-O-Acetyl-4''-O-[(R)-(-)-O-chloroacetyl-p-methoxymandeloyl]-4'''-O-trimethylsilytylosin (29.3 mg, 0.0233 mmol) was dissolved in 1.5 ml of methanol, and then 0.15 ml of water was added and the solution was heated under reflux for 9 hours. After the reaction mixture was cooled to room temperature, 1.2 ml of 0.025N HCl was added to the mixture and stirring was continued for 30 minutes. A small amount of chloroform and 2 ml of saturated aqueous sodium bicarbonate were added to the mixture, and then the mixture was extracted with chloroform. The chloroform layer was washed with a saturated sodium chloride solution, dried over anhydrous sodium sulfate, and the solvent was evaporated under reduced presssure. The residue obtained was purified by preparative thin layer chromatography (developer: chloroform/methanol = 10/1: v/v) to give 21.1 mg of 4''-O-[(R)-(-)-p-methoxymandeloyl]tylosin (yield: 83.3%).

The physical properties of the title compound are as follows:

m.p. 121.0-124.5 ° C $[\alpha]_D^{22}$ -38.4 ° (c 1.0, methanol)

$$UV \quad \lambda_{max}^{MeOH} \quad (nm):$$

282.3($\epsilon$ 24800), 232.5($\epsilon$ 12500)

$$IR \quad \nu_{max}^{CHCl_3} \quad (cm^{-1}):$$

3470, 2920, 1720, 1675, 1590, 1510, 1165, 1080

$^1$H-NMR ($CDCl_3$, $\delta$ ppm):

0.58(3H,d,J = 6.2Hz,H-6''), 1.80(3H,s,12-$CH_3$), 2.41(6H,s,3'-N$(CH_3)_2$), 3.49(3H,s,2'''-$OCH_3$), 3.62(3H,s,3'''-$OCH_3$), 3.80(3H,s,$CH_3O$-$C_6H_4$-), 4.21(1H,d,J = 7.3Hz,H-1'), 4.55(1H,d,J = 10.3Hz,H-4''), 4.5 6-(1H,d,J = 7.7Hz,H-1'''), 5.04(1H,d,J = 3.3Hz,H-1''), 5.28(1H,s,-$C_6H_4$-CH(OH) -CO), 5.91(1H,d,J = 10.6Hz, H-13), 6.26(1H, d,J = 15.4Hz,H-10), 6.87(2H,d,J = 8.8Hz,

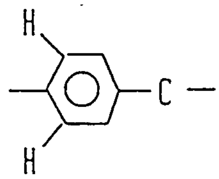

),

7.32(1H,d,J = 15.4Hz,H-11), 7.40(2H,d,J = 8.8Hz,

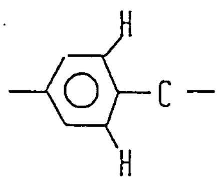

),

9.67(1H,s,CHO)

Example 3

(a) Preparation of (S)-(+)-O-chloroacetyl-p-methoxymandelic acid.

(S)-(+)-p-methoxymandelic acid (184 mg, 1.01 mmol), obtained according to the method described in Example 1, was dissolved in 4 ml of tetrahydrofuran and the solution was cooled to -20° C. After 0.205 ml of pyridine (2.53 mmol) was added, 0.179 ml of chloroacetyl chloride (2.25 mmol) was gradually added to the solution and the mixture was stirred at -20 ° C for 1 hours. Toluene (5 ml) and water (5 ml) were added to the reaction mixture, and then the reaction mixture was warmed up to room temperature. The pH of the reaction mixture was adjusted to 2 with 1N HCl to separate toluene layer, and the toluene layer was washed with water, dried over anhydrous sodium sulfate, and then the solvent was evaporated under reduced pressure. The residue obtained was applied on column chromatography on 15 g silica gel (eluent: chloroform/methanol = 50/1 :v/v) to give 173 mg of (S)-(+)-O-chloroacetyl-p-methoxymandelic acid (yield: 66.2%).

The physical properties of the title compound are as follows:
$[\alpha]_D^{25}$ +140° (c 0.95, acetone)

IR $\nu_{max}^{CHCl_3}$ ($cm^{-1}$):

1740, 1610, 1510, 1245, 1165, 1030, 830
$^1$H-NMR ($CDCl_3$, δ ppm):
3.81(3H,s,$OCH_3$), 4.17(2H,ABq,J = 14.7Hz,$COCH_2Cl$), 5.96(1H,s,-$C_6H_4$-CH(OH) -CO), 6.92(2H,d,J = 8.8Hz,

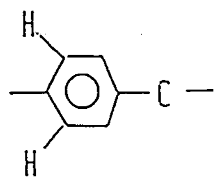

),

7.38(2H,d,J = 8.8Hz,

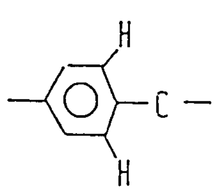

),

9.41(1H,brs, COOH)

(b) Preparation of 2'-O-acetyl-4''-O-[(S)-(+)-O-chloroacetyl-p-methoxymandeloyl]-4'''-O-trimethylsilyltylosin

(S)-(+)-O-Chloroacetyl-p-methoxymandelic acid (102 mg, 0.394 mmol) was dissolved in 1.5 ml of 1,2-dichloroethane. The solution was cooled to -30 °C and then 0.032 ml of pyridine (0.39 mmol) and 0.050 ml of pivaloyl chloride (0.41 mmol) were added to the solution and the mixture was stirred at -30°C . After 15 minutes, 101 mg of 2'-O-acetyl-4'''-O-trimethylsilyltylosin (0.0980 mmol), 0.068 ml of triethylamine (0.49 mmol), and 3.6 mg of 4-dimethylaminopyridine (0.029 mmol) were added to the mixture, and stirring was continued at the same temperature for 2 hours. A small amount of chloroform and 5 ml of water were added to the reaction mixture, and then the mixture was warmed to room temperature to separate an organic layer. The separated organic layer was washed with saturated aqueous sodium bicarbonate and then with a saturated sodium chloride solution, dried over anhydrous sodium sulfate, and the solvent was evaporated. The residue was applied on column chromatography on 10 g silica gel (eluent: toluene/acetone = 8/1 and then 7/1: v/v) to give 69.5 mg of 2'-O-acetyl-4''-O-[(S)-(+)-O-chloroacetyl-p-methoxymandeloyl]-4'''-O-trimethylsilyltylosin (yield: 56.4%).

The physical properties of the title compound are as follows:
m.p. 115.5-117.0°C

$$UV\ \lambda_{max}^{MeOH}\ (nm):$$

281.0(ε 20700), 234.9(ε 13200)

$$IR\ \nu_{max}^{CHCl_3}\ (cm^{-1}):$$

3470, 2920, 1740, 1715, 1670, 1605, 1585, 1505, 1245, 1160, 1095, 875, 835
$^1$H-NMR ($CDCl_3$, δ ppm):
0.17(9H,s,$(CH_3)$,Si), 0.71(3H,s,3''-$CH_3$), 1.79(3H,s,12-$CH_3$), 2.08(3H,s,2'-OAc), 2.42(6H,s,3'-$N(CH_3)_2$). 3.50-(3H,s,2'''-$OCH_3$), 3.60(3H,s,3'''-$OCH_3$), 3.80(3H,s,$CH_3O$-$C_6H_4$-), 5.93(1H,s,-$C_6H_4$-CH(OH)-CO), 5.94-(1H,d,J = 11.0Hz,H-13), 6.29(1H,d,J = 15.4Hz,H-10), 6.91(2H,d,J = 8.8Hz,

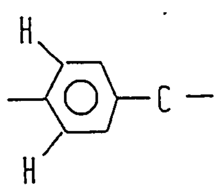
),

7.32(1H,d,J = 15.4Hz,H-11), 7.43(2H,d,J = 8.8Hz,

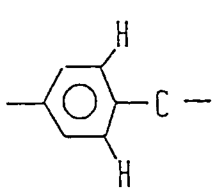
),

9.66(1H,s,CHO)

(c) Preparation of 4''-O-[(S)-(+)-p-methoxymandeloyl]tylosin (Compound A)

2'-O-Acetyl-4''-O-[(S)-(+)-O-chloroacetyl-p-m ethoxymandeloyl]-4'''-O-trimethylsilyltylosin (69.5 mg, 0.0554 mmol) was dissolved in 3 ml of methanol, and then 0.3 ml of water was added and the solution was heated under reflux for 8 hours. After the reaction mixture was cooled to room temperature, 2.5 ml of 0.025N HCl was added to the mixture and stirring was continued for 30 minutes. A small amount of chloroform and 2 ml of saturated aqueous sodium bicarbonate were added to the mixture, and then the mixture was extracted with chloroform. The chloroform layer was washed with a saturated sodium chloride solution, dried over anhydrous sodium sulfate, and the solvent was evaporated under reduced presssure. The residue obtained was purified by preparative thin layer chromatography (developer: chloroform/methanol = 10/1: v/v) to give 40.7 mg of 4''-O-[(S)-(+)-p-methoxymandeloyl]tylosin (yield: 68.0%).

The physical properties of the title compound are as follows:
m.p. 112.0-115.0 °C $[\alpha]_D^{22}$ -37.1 ° (c 1.03, methanol)

UV $\lambda_{max}^{MeOH}$ (nm):

281.7($\epsilon$ 25300), 232.4($\epsilon$ 12600)

IR $\nu_{max}^{CHCl_3}$ ($cm^{-1}$):

3450, 2910, 1715, 1670, 1585, 1505, 1160, 1070, 830
$^1$H-NMR ($CDCl_3$, $\delta$ ppm):
0.80(3H,s,3''-$CH_3$), 1.79(3H,s,12-$CH_3$), 2.51(6H,s,3'-N$(CH_3)_2$), 3.49(3H,s,2'''-$OCH_3$), 3.61(3H,5,3'''-$OCH_3$), 3.79(3H,s,$CH_3O$-$C_6H_4$-), 4.21(1H,d,J=7.7Hz,H-1'), 4.56(1H,d,J=7.7Hz,H-1'''), 4.5 8(1H,d,J=10.3Hz,H-4''), 5.04(1H,d,J=3.3Hz,H-1''), 5.22(1H,s,-$C_6H_4$-CH(OH)-CO), 5.91(1H,d,J=10.3Hz,H-13), 6.27(1H,d,J=15.4Hz,H-10), 6.88(2H,d,J=8.8Hz,

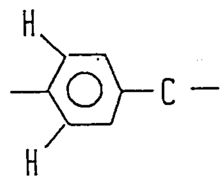

),

7.32(1H,d,J=15.4Hz,H-11), 7.41(2H,d,J=8.8Hz,

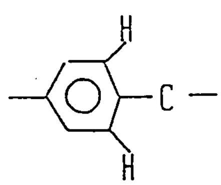

),

9.67(1H,s,CHO)

Example 4

a) Preparation of (S)-O-tetrahydropyranyl-p-methoxymandelic acid

(S)-p-Methoxymandelic acid (8.74 g, 48 mmol) was dissolved in 100 ml of tetrahydrofuran, and 5.69 ml of 3,4-dihydro-2H-pyrane (62.4 mmol) was added to the solution. Under ice cooling, 500 mg of p-toluenesulfonic acid was added to the solution, and the mixture was stirred at 0 °C for 2 hours and then at 25 °C for 3 hours. After saturated aqueous sodium bicarbonate solution was added to the reaction mixture

to neutralize the solution, most of tetrahydrofuran was evaporated under reduced pressure. Ethyl acetate was added to the concentrated reaction mixture, and then the mixture was adjusted to pH 3.0 by adding 0.3N aqueous hydrochloric acid and extracted. The organic layer was washed with water and dried over anhydrous sodium sulfate. After being filtrated, the organic layer was evaporated under reduced pressure to give 14.0 g of syrup. The syrup was chromatographed on a 150 g silica gel column (eluent: chloroform/methanol = 30/1), and the fractions containing the title compound, which were detected by UV absorption at Rf = 0.54 on a silica gel TLC plate (developer: chloroform/methanol = 10/1), were collected and concentrated to give 10.8 g of (S)-O-tetrahydropyranyl-p-methoxymandelic acid (yield: 85%).

The physical properties of the title compound are as follows:

$[\alpha]_D^{25}$ +61.2° (c 1.0, acetone)

$$IR \quad \nu_{max}^{KBr} \quad (cm^{-1}):$$

3468, 1728, 1514, 1248, 1034

$^1$H-NMR ($CDCl_3$, δ ppm):

1.45-1.93(6H,m,$CH_2$-$CH_2$-$CH_2$), 3.45-4.05(2H,m,$CH_2$-O), 3.80(3H,s,$OCH_3$), 4.58(0.5H,t,J = 3.9Hz,O-CH-O), 4.86(0.5H,dd,J = 2.9Hz,3.2Hz,O-CH-O), 5.20(0.5H,s,O-CH-COO), 5.28(0.5H,s,O-CH-COO), 6.889-(1H,d,J = 8.8Hz,Ar), 6.894(1H,d,J = 8.8Hz,Ar), 7.35(1H,d,J = 8.8Hz,Ar), 7.41(1H,d,J = 8.8Hz,Ar)

(b) Preparation of 2'-O-acetyl-4''-O[(S)-(+)-O-tetrahydropyranyl-p-methoxymandeloyl]-4'''-O-trimethylsilyltylosin

After 320 mg of (S)-O-tetrahydropyranyl-p-methoxymandelic acid (1.21 mmol), prepared in the above procedure, was dissolved in 3 ml of ethyl acetate, 146.9 mg of dicyclohexylcarbodiimide (0.712 mmol) was added to the solution and then the mixture was stirred at 0°C for 20 minutes. To the mixutre, 117 µl of triethylamine (0.846 mmol), 14.8 mg of dimethylaminopyridine (0.12 mmol), and 373 mg of 2'-O-acetyl-4'''-O-trimethylsilyltylosin (0.363 mmol) were added and the resulting mixture was sitrred for 16 hours at room temperature. After filtration, ethyl acetate was added to the mother liquor to obtain a solution in a total volume of 20 ml, and the solution was washed with a saturated aqueous sodium bicarbonate solution, saturated sodium chloride solution, and then with water. The organic layer was dried over anhydrous sodium sulfate and evaporated under reduced pressure. The residue was chromatographed on a 50 g silica gel column (eluent: toluene/acetone = 7/1), and the fractions containing the title compound, which were detected by UV absorption at Rf = 0.62 on a silica gel TLC plate (developer: toluene/acetone = 3/1), were collected and concentrated to give 140 mg of 2'-O-acetyl-4''-O-[(S)-(+)-O-tetrahydropyranyl-p-methoxymandeloyl]-4'''-O-trimethylsilyltylosin (white powder, yield: 30%).

The physical properties of the title compound are as follows:

$$IR \quad \nu_{max}^{KBr} \quad (cm^{-1}):$$

3515, 2938, 1748, 1593, 1373, 1250, 1100, 1059

$^1$H-NMR ($CDCl_3$, δ ppm):

0.17(9H,s,$(CH_3)_3$Si), 0.77(3H,s,H-7''), 1.78(3H,s,12-$CH_3$), 2.08(3H,s,2'-OAc), 2.41(6H,s,$(NCH_3)_2$), 3.50-(3H,s,2'''-$OCH_3$), 3.60(3H,s,3'''-$OCH_3$), 3.79(3H,s,$CH_3$O-Ar), 4.26(1H,d,J = 7.3Hz,1'-H), 4.37(1H,m,5''-H), 4.61-(0.5H,m,THP-anomeric), 4.88(0.5H, brs,THP-anomeric), 5.20(0.5H,s,O-CH-COO), 5.34(0.5H,s,O-CH-COO), 5.94(1H,d,J = 10.3Hz,H-13), 6.28(1H,d,J = 15.4Hz,H-10), 6.87(2H,d,J = 8.8Hz,Ar), 7.32(1H,d,J = 15.4Hz,H-11), 7.43(1H,d,J = 8.8Hz,Ar), 7.4 7(1H,d,J = 8.8Hz,Ar), 9.66(1H,s,CHO)

(c) Preparation of 4''-O-[(S)-(+)-p-methoxymandeloyl]tylosin

2'-O-Acetyl-4''-O-[(S)-(+)-O-tetrahydropiranyl-p-methoxymandeloyl]-4'''-O-trimethylsilyltylosin (29.5 mg, 0.0232 mmol), obtained in the above procecure, was dissolved in 2 ml of methanol and 0.2 ml of water was added to the solution. The mixture was heated at 80 °C under reflux for 13 hours. After concentration of the mixture, 2 ml of acetone, 0.5 ml of water, and 0.8 ml of 0.1N aqueous hydrochloric acid was added to the concentrate and the mixture was stirred for 3.5 hours at room temperature. A saturated aqueous sodium

bicarbononate solution was added to neutralize the reaction mixutre, and 30 ml of ethyl acetate was added to extract the mixture. The organic layer was washed with water and dried over anhydrous sodium sulfate. After being filtered, the organic layer was concentrated under reduced pressure, and the residue was chromatographed by preparative thin layer chromatography (developer: toluene/acetone = 1/1), and the zone containing the title compound, which was detected by UV absorption at Rf = 0.45, was collected and concentrated to give 14 mg of 4"-O-[(S)-(+)-p-methoxymandeloyl]tylosin(yield: 56 %). The physical properties of the compound obtained were identical with compound B obtained in Example 3.

One of ordinary skill in the art will recognize that improvements and modifications may be made while remaining within the scope of the present invention. The scope and spirit of the present invention is determined soley by the appended claims.

## Claims

**1.** A 4"-O-p-methoxymandeloyltylosin compound represented by the following formula:

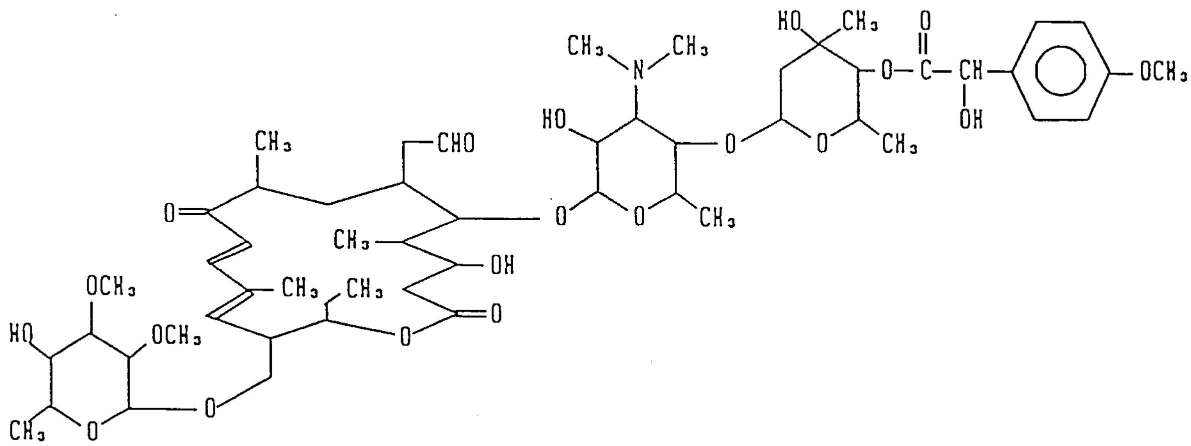

**2.** A compound according to claim 1, being 4"-O-[(S)-(+)-p-methoxymandeloyl]tylosin.

**3.** A compound according to claim 1, being 4"-O-[(R)-(-)-p-methoxymandeloyl]tylosin.

**4.** A compound according to claim 1, being 4"-O-[(±)-p-methoxymandeloyl]tylosin.

**5.** A method for preparing 4"-O-p-methoxymandeloyltylosin which comprises the steps of:
(a) condensing a protected para-methoxymandelic acid having a protected hydroxy group with tylosin or a tylosin derivative having one or more protective groups; and
(b) removing the protective group to obtain 4"-O-p-met hoxymandeloyltylosin.

**6.** A method for preparing 4"-O-p-methoxymandeloyltylosin according to claim 5, wherein the protected para-methoxymandelic acid is selected from the group consisting of protected (-)-para-methoxymandelic acid, protected (+)-para-methoxymandelic acid, and protected (±)-para-methoxymandelic acid.

**7.** A method for preparing 4"-O-p-methoxymandeloyltylosin according to claim 5, wherein the protected tylosin is 2'-O-acetyl-4'''-O-trimethylsilyltylosin.

**8.** A pharmaceutical composition comprising an effective amount of 4"-O-p-methoxymandeloyltylosin and a pharmaceutically acceptable carrier or coating.

**9.** A method for treating an infectious disease which comprises administering to an animal an effective amount of the compound 4"-O-p-methoxymandeloyltylosin.

**10.** A method according to claim 9, comprising administering a pharmaceutical composition comprising said compound together with a pharmaceutically acceptable carrier or coating.

European Patent Office

# EUROPEAN SEARCH REPORT

Application Number

**EP 91 11 8020**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 001 841 (SANRAKU OCEAN CO. LTD.)<br>* abstract *<br>* page 1, line 1 - page 5, line 23 * * | 1,5,8-10 | C 07 H 17/08<br>A 61 K 31/70 |
| A | EP-A-0 145 023 (KYOWA HAKKO KOGYO CO. LTD.)<br>* abstract * * | 1,5,8-10 | |
| A | EP-A-0 287 082 (SOUR PLIVA FARMACEUTSKA, KEMIJSKA PREHRAMBENA I KOZMETICKA INDUSTRIJA)<br>* abstract * * | 1,5,8-10 | |

TECHNICAL FIELDS SEARCHED (Int. Cl.5)

C 07 H
A 61 K

The present search report has been drawn up for all claims

| Place of search | Date of completion of search | Examiner |
|---|---|---|
| The Hague | 16 December 91 | SCOTT J.R.M. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document